Europäisches Patentamt

European Patent Office

Office européen des brevets

(11) Publication number: **0 077 196**
A2

(12) **EUROPEAN PATENT APPLICATION**

(21) Application number: 82305375.6

(22) Date of filing: 08.10.82

(51) Int. Cl.³: **C 12 N 15/00**, C 12 N 9/00, C 12 P 13/22, C 12 N 1/00 // C12R1/19

(30) Priority: 09.10.81 US 310368
29.09.82 US 427443

(43) Date of publication of application: 20.04.83
Bulletin 83/16

(84) Designated Contracting States: **AT BE CH DE FR GB IT LI LU NL SE**

(71) Applicant: **GENEX CORPORATION, 6110 Executive Boulevard, Rockville Maryland 20852 (US)**

(72) Inventor: **Synenki, Richard A., 210 Gold Kettle Drive, Gaithersburg Maryland 20878 (US)**

(74) Representative: **Holmes, Michael John et al, Frank B. Dehn & Co. Imperial House 15-19 Kingsway, London WC2B 6UZ (GB)**

(54) **Aromatic amino acid-producing microorganisms.**

㊸ An isolated microbial gene that specifies the biosynthesis of a 3-deoxy-2-keto-D-arabinoheptulosonic acid-7-phosphate (DAHP) synthetase, which is resistant to feedback inhibition by aromatic amino acids; plasmids containing such a gene; and microorganisms transformed thereby.

EP 0 077 196 A2

## AROMATIC AMINO ACID-PRODUCING MICRO-ORGANISMS

The present invention relates to the microbial production of aromatic amino acids. More particularly, the invention concerns the genetic modification of microorganisms to enhance their ability to efficiently produce aromatic amino acids.

The aromatic amino acids, tyrosine, tryptophan and phenylalanine, are essential nutrients for humans and most animals. These compounds have become important items of commerce, because of their use in hyperalimentation formulations, nutritional supplements and as intermediates for other useful products.

Production of aromatic amino acids is presently accomplished largely by fermentive methods employing tyrosine-, tryptophan- or phenylalanine-producing strains of bacteria. The bacterial biosynthesis of all three amino acids involves a common metabolic pathway that begins with the precursors, erythrose-4-phosphate (E-4-P) and phosphoenolpyruvate (PEP). These precursors combine to form an intermediate, 3-deoxy-D-arabinoheptulosonic acid-7-phosphate (DAHP). The metabolic pathway continues from DAHP through a number of intermediates including shikimic acid to chorismic acid, which is the last common intermediate for the three aromatic amino acids. Chorismic acid is, in turn, converted to anthranilic acid, which is an intermediate for tryptophan and to prephenic acid, which is an intermediate for phenylalanine and tyrosine.

The first step of the metabolic pathway (reaction of E-4-P and PEP to form DAHP) is catalyzed by three isoenzymes, collectively known as DAHP synthetase. The biosynthesis of each of these isoenzymes is controlled by feedback inhibition by one of the three products, tyrosine, tryptophan and phenylalanine. Other enzymatic reactions of the metabolic pathway may also be controlled by feedback inhibition by the final products or by intermediate products. Feedback inhibition is the natural means by which an organism prevents the overproduction of certain compounds, but in an industrial setting, feedback inhibition limits fermentation efficiencies and the total amount of product that can be recovered.

Feedback resistant strains of aromatic amino acid-producing microorganisms have been isolated from wild-type cultures and have also been obtained by conventional mutation and selection prodecures. Held, W. A. and Smith, O. H., Journal of Bacteriology, 101, No. 1, 202-208 (1970). Overall fermentation efficiencies have been improved using such strains; however, it has not heretofore been possible to combine feedback resistance mutations with other desirable culture characteristics in a single microorganism.

Genetic engineering techniques offer the possibility of introducing genes that specify feedback resistant enzymes into the genomes of aromatic amino acid-producing cells. The E.coli genes that code for the DAHP synthetase isoenzymes are known as aroF, aroG and aroH. Held and Smith (supra.) describe an E. coli mutant (MAR13) derived from strain W1485 that possesses an aroG gene for DAHP synthetase which is resistant to feedback inhibition by phenylalanine. A sample of this strain has been deposited with the American Type Culture Collection, Rockville, Maryland, as ATCC 31993.

## SUMMARY OF THE INVENTION

In accordance with the present invention, an isolated, microbial gene that specifies the biosynthesis of a 2-deoxy-2-keto-D-arabinoheptulosonic acid-7-phosphate (DAHP) synthetase, which is resistant to feedback inhibition by aromatic amino acids. In other aspects, the invention involves a plasmid; in which such a feedback resistant gene is cloned, and a microorganism transformed by such a plasmid.

BRIEF DESCRIPTION OF THE DRAWINGS

Figure 1 is the nucleotide sequence of a. DAHP. synthetase gene of the present invention;

Figure 2 is a restriction map and diagram of a plasmid containing the DAHP synthetase gene of the present invention.

DETAILED DESCRIPTION OF THE INVENTION

As a source of a feedback resistant aroF, aroG or aroH gene for cloning, a mutant strain is selected, which produces DAHP synthetase in the presence of relatively high concentrations of aromatic amino acids. Various such strains are known (e.g., see Held; W.A., et al., supra.) or can be produced by well known artificial mutation and selection procedures. E.coli strains are convenient sources of such genes, because the genetics of that organism have been studied extensively and are relatively well understood. However, any microorganism that utilizes the DAHP-shikimic acid pathway can be employed as a potential source of the feedback resistant gene.

The preparation of a genetically modified organism that contains a cloned feedback resistant DAHP synthetase gene involves several steps. As an initial step, the gene is isolated from the feedback resistant mutant and is cloned into a cloning vector. Cells are then transformed with the cloning vector to amplify the gene, and transformants are selected by means of selectable markers or on the basis of the cells' ability to produce DAHP-synthetase activity which is not inhibited by the presence of aromatic amino acids or analogues thereof. After amplifying the gene, it may be excised from the cloning vector and inserted into an efficient expression vector, which is then used to transform aromatic amino acid-producing host cells.

Isolation of a feedback resistant DAHP synthetase gene from a feedback resistant mutant is accomplished using well known procedures. Generally, the cells are disrupted, e.g., by mechanical homogenation and/or enzymatic lysis, and the solid cellular debris is removed by centrifugation. Proteins may be precipitated from the cytoplasm with phenol, and removed by centrifugation, and the DNA can then be recovered by precipitation with ethanol.

The total cellular DNA is then digested with a restriction endonuclease to provide a mixture of restriction fragments of varying sizes. Partial restriction digestion with such restriction endonuclease is preferred, because partial digestion increases the probability that fragments will be generated that contain the intact DAHP synthetase gene of interest (i.e., feedback resistant aroF, aroG, or aroH.). Any restriction endonuclease having a recognition sequence which occurs frequently in microbial DNA may be employed for the digestion. EcoRI is a preferred restriction endonuclease, both because its recognition sequence is common in microbial DNA, and because EcoRI sites are convenient insertion sites in cloning vectors subsequently used for amplification of the gene.

The restriction fragments resulting from the digestion reaction are advantageously subjected to a sizing technique to isolate larger fragments likely to contain the gene of interest in full length. Conventional sizing techniques, such as electrophoresis, or density gradient centrifugation can be employed, and sucrose density gradient centrifugation is a particularly preferred technique. Although the DAHP synthetase gene is thought to have a size of about 1.2 kb, the probability of obtaining fragments containing the intact gene is improved by selecting larger fragments, for example of about 2 kb or larger, and preferably larger than about 10 kb. On the other hand, large fragments, when inserted into a cloning vector, can produce unnecessarily large recombinant molecules. Accordingly, restriction fragments smaller than about 35 kb are advantageously employed.

Preferably, a restriction fragment containing the feedback resistant gene of interest along with a phenotypic marker can be obtained. For example, a 31 kb fragment isolated from the total DNA of the MAR13 strain of E.coli (supra.), has been found to contain the galactose operon (gal) in proximity to a feedback resistant aroG gene. Cells that contain the gal operon may be easily scored by their ability to produce a red pigment on indicator plates, such as MacConkey's media containing galactose. Therefore, the gal operon can be used as a phenotypic marker to select transformants likely to contain the aroG gene.

It has been found that conducting the cloning phase in two steps, as hereinafter described, results in a higher frequency of transformants containing the cloned gene of interest. The sized restriction fragments are advantageously inserted first into a single copy plasmid, i.e., one which exhibits strictly controlled replication and generally exists in vivo at a frequency of only one molecule per cell. Using a single copy plasmid is thought to enhance the chances for successfully cloning a larger fragment containing numerous known and unknown genes. A particularly preferred cloning vector is the single copy plasmid pMF 3. This plasmid is isolated from E.coli strain BK262 (Cold Spring Harbor No. 50), which is described by Madison and Kline, MGG, 152, 175-182 (1977). A sample of this strain has been deposited with the American Type Culture Collection, Rockville, Maryland, as ATCC 31994.

To prepare recombinant plasmids that potentially contain a restriction fragment having the feedback resistant DAHP synthetase gene, the restriction fragments are incubated with restriction digested plasmids in the presence of DNA ligase, under annealing and ligating conditions. Joining of the restriction fragments to the linearized plasmids can be accomplished by any of a number of known procedures. For example, the preferred procedure involves cutting the plasmid with the same restriction endonuclease used for preparing the restriction fragments. Alternatively, complementary homopolymeric tails (e.g., poly d[C] and poly d[T]) can be ligated to the 5' ends of the restriction fragments and plasmid, respectively. Jackson, et al., Proc. Natl. Acad. Sci. USA, 69, 2904-2909. (1972). Other techniques, such as 5'-exonuclease treatment, followed by blunt-end ligation, or the use of short segments of DNA (oligonucleotide linkers) may be employed, if desired.

The frequency of cloning a restriction fragment can be improved by first treating the linearized plasmid DNA to remove the 5' phosphate groups. The removal of these groups prevent recircularization of the plasmid or the formation of dimers, trimers and the like. The phosphate groups are advantageously removed by treating the DNA with an alkaline phosphatase, such as that derived from calf intestines. Efstratiadis, et al., NAR 4(12), 4165-4174 (1977).

After ligating restriction fragments into a cloning vector, the recombinant plasmids are used to transform host cells. E.coli cells are preferably used for this purpose, and other bacterial cells, with which the cloning vector is compatible, may also be employed. The cells can be transformed by conventional procedures involving treatment with calcium ions to make them competent to accept foreign DNA. Cohen, S.N., et al., PNAS 69, 2110-2114 (1972). Transformed cells are then plated on a nutrient medium designed for selection of transformants based on phenotypic properties specified by the recombinant plasmid of interest. For example, in selecting the transformants containing the MAR13 fragment having the gal gene, nutrient media supplemented with galactose may be advantageously employed. Similarly, media containing various antibiotics or growth factors can be used, depending upon the composition of the cloning vector.

Further selection may be made on the basis of the size of plasmids in the transformant cells, inasmuch as the sizes of the original plasmid and the restriction fragment inserts are known.

If the initial cloning was accomplished using a single copy plasmid, as described above, the gene is preferably subcloned in a multi-copy plasmid (i.e., one that exhibits a relaxed mode of replication). To accomplish the subcloning, transformants having the cloned feedback resistant DAHP synthetase gene are cultivated on a growth medium to grow an ample quantity of cells for subsequent manipulations. The plasmid DNA is recovered from these cells and isolated, and the DAHP synthetase gene is excised by digestion with a restriction endonuclease. Preferably, the same restriction endonuclease that was used for digesting the cellular DNA is employed for excising the DAHP synthetase gene. The gene is then isolated, e.g., by sucrose gradient centrifugation, and is inserted into a multi-copy plasmid. The insertion is accomplished by known techniques, substantially as described above in connection with the single copy plasmid. A variety of such multi-copy plasmids may be employed, and the well known plasmid designated pBR322 is preferred. The pBR322 plasmid has an EcoRI restriction site and, therefore, genes that have been excised with EcoRI may conveniently be inserted into the pBR322 plasmid by a simple ligation procedure.

The recombinant plasmids are again used to transform host cells, and transformants containing the feedback resistant DAHP synthetase gene are selected on the basis of phenotypic markers on the plasmid, the gene insert, and on the basis of size.

Before or subsequent to cloning the DNA fragment containing the feedback resistant DAHP synthetase gene into a multi-copy plasmid, steps are advantageously taken to reduce the size of this fragment. For example, it has been found that the aroG gene is contained within a fragment that can be isolated by restriction with the endonuclease Hind III. Unexpectedly, it has been found that the Hind III restriction fragment from the aroG gene from MAR13 is further reduced in vivo in E.Coli to a fragment containing about 8.2 kb. Although this internal deletion reduces the size of the Hind III fragment by about 50%, it has not been found to delete any portion of the aroG gene. Further reductions in the size of the cloned DNA fragment may be accomplished by selective restriction endonuclease digestions. A SalI-Hind III digestion, for example, has been used to reduce the size of the aroG fragment from MAR13 to about 3 kb.

In addition to the DAHP synthetase gene, the cloned DNA fragment also advantageously includes nucleotide sequences that control the expression of the gene. These sequences permit expression of the gene (e.g., production of a DAHP synthetase enzyme) by cells transformed by plasmids into which the gene has been cloned. Alternatively, such control signals may be provided as a part of the vector into which the gene is cloned, or may be spliced onto the gene prior to insertion into the vector.

E.coli cells obtained in this manner have been found to produce significantly greater than normal amounts of DAHP synthetase. Moreover, the DAHP synthetase so produced is active in the presence of high concentrations of aromatic amino acids. Having cloned the feedback resistant DAHP synthetase gene, this gene can be selectively combined with host strains and other genetic modifications to produce highly efficient aromatic amino acid-producing strains. Because of the degeneracy of the genetic code, the nucleotide sequence of the DAHP gene shown in the drawing can be varied substantially, without changing the amino acid sequence of the expressed protein. Accordingly, having described the gene, its nucleotide and amino acid sequence, and procedures for its isolation and cloning, the present invention encompasses the variations of the nucleotide sequence permitted by the genetic code.

The invention is further illustrated by the following examples which are not intended to be limiting.

EXAMPLE I

Strain Wl485 (MAR13) was grown to stationary phase in L broth by incubating for 18 hours at 37°C. The cells were isolated, washed and lysed. The DNA was isolated by ethanol precipitation after the lysed solution was phenol and chloroform extracted. After ethanol precipitation, the DNA was spooled 3 times and resuspended in TE buffer (Marmur, J., 1961, J. Mol. Biol., 3, 208-218). 80 ug of MAR13 DNA was digested for 2 hours at 37°C. with 40 units of EcoRI restriction enzyme. This DNA was placed on a 15-32% logarithmic sucrose gradient and centrifuged for 18 hours at 37°C. at 4°C. The 3 fractions containing the largest DNA fragment were pooled, the DNA ethanol precipitated, and this DNA (8 ug) used for cloning the aroG gene.

EXAMPLE II

Restriction enzyme digested fragments purified on a sucrose gradient having a size of about 30 kb, prepared as described in Example I were inserted into plasmid pMF-3 by the following procedure: Approximately 14 ug of pMF-3 plasmid was digested with 27 units of EcoRI endonuclease for an hour. These mixtures were then heated at 65°C. for 15 minutes to terminate the endonuclease reaction, and the plasmid DNA was precipitated by the addition of ethanol in an isopropanol/dry ice bath. The linearized plasmid DNA was then treated with alkaline phosphatase to remove the 5' phosphate groups. The alkaline phosphatase was prepared as follows:

10 microliters of a calf alkaline phosphatase solution (purchased from Boehringer Mannheim, Indianapolis, Indiana) were centrifuged for 15 minutes at 15,000 rpm at about 0°C. The solutions were then removed from the centrifuge, the supernatant removed with filter paper, and the enzyme pellet was resuspended in 100 ml of glycine buffer. This suspension was then diluted to 4.9 ml with glycine buffer and the preparation was left on ice for 15 minutes.

The linearized plasmid pellets were then resuspended in 80 microliters of TE buffer, and to one-half of this suspension was added 10 microliters of the calf alkaline phosphatase preparation. The solution was then incubated for 3 hours at 37°C. The DNA from the solution was then precipitated with ethanol, centrifuged, and the pellet was stored at -20°C.

Three fractions of the sucrose gradient-purified MAR13 DNA were pooled (as described in Example I) and resuspended in 56 microliters of 10 mM tris-6mM $MgCl_2$ (pH 7.4). The treated plasmid DNA was then suspended in 14 microliters of the same tris-$MgCl_2$ buffer and was added to 14 microliters of the MAR13 DNA suspension. To this mixture was added 1.0 microliter of 1M $MgCl_2$, 1.0 microliter of 10 mM dithiothreitol and 0.5 microliters of 10 mM ATP. Five microliters of this reaction mixture were removed for electrophoresis on a 0.7% Agarose gel as a control for unligated material. To the remainder of the mixture was added 1 microliter of a 1:10 dilution of T4-DNA ligase (purchased from Bethesda Research Laboratories, Rockville, MD). The ligation mixture was then incubated at 12°C. for 2 hours. Five microliters of the reaction mixture was again removed from Agarose gel electrophoresis to monitor the degree of ligation. To the remaining 20 microliters of the reaction mixture was added 80 microliters of a solution containing the following:

262 microliters of 10 mM tris-6mM $MgCl_2$ (pH 7.4)

10 microliters 1M $MgCl_2^-$

7 microliters in 10 mM dithiothreitol

5 microliters 10 mM ATP

36 microliters of 1:10 T4 ligase dilution

This mixture was then incubated at 12° C for 18 hours. The recombinant plasmids prepared in this manner were used to transform E.coli cells as follows:

The host cells were from a strain which has a deletion for the (Gal-aroG) region. A sample of this strain has been deposited with the American Type Culture Collection, Rockville, Maryland, as ATCC 31995. A culture of the cells was prepared by inoculating 100 milliliters of fresh LB broth with 1 milliliter of a cell culture, and the medium was incubated at 37°C with shaking to an optical density (590nm) of 0.22. The cells were harvested, washed in ice-cold 100 mM $MgCl_2$, and resuspended in 40 milliliters of ice-cold 100 mM $CaCl_2$-50mM $MgCl_2$ and held on ice for 90 minutes. The cells were resuspended in 1 milliliter of cold 100 mM $CaCl_2$50mM $MgCl_2$ and stored at 4°C. for 20 hours. The cells were then heat shocked at 42°C. for three minutes, 1 milliliter of LB broth supplemented with 2% galactose was added, and the mixture was incubated at 37°C. with shaking for 90 minutes. Fifty microliters of this incubation mix was plated 20 ug/ml of MacConkey's medium supplemented with galactose and ampicillin. From a total of 308 colonies, 15 of those were red. Several of the galactose positive colonies were cultivated in liquid broth and analyzed for DAHP synthetase activity until the colony having AroG feedback resistant activity was found. DAHP synthetase activity was determined by the method of MacCandliss, R.J., et al., J. Biol. Chem., 253, No.12, 4259-4265 (1978).

EXAMPLE III

Supercoiled plasmid DNA was isolated from the feedback resistant aroG clone prepared as described in Example II by a standard plasmid isolation procedure described by Humphreys, et al., BBA, 383:457 (1975) and Guerry, et al., J. Bact., 116:1064 (1973). One microgram of this plasmid was digested with Hind III restriction enzyme and ligated with two micrograms of pBR322 DNA, which had also been cut at its Hind III site. These DNA's were ligated, cells were transformed, and cells containing the cloned feedback resistant aroG gene were selected essentially as described in Example II. Of all of the colonies which appeared, only one was red (i.e., galactose positive). This colony was cultivated in liquid broth assayed for feedback resistant aroG DAHP synthetase activity, which confirmed that such activity was present. Analysis of the plasmid content of these cells indicated that a deletion of about 7.1 kb of the plasmid insert had occurred.

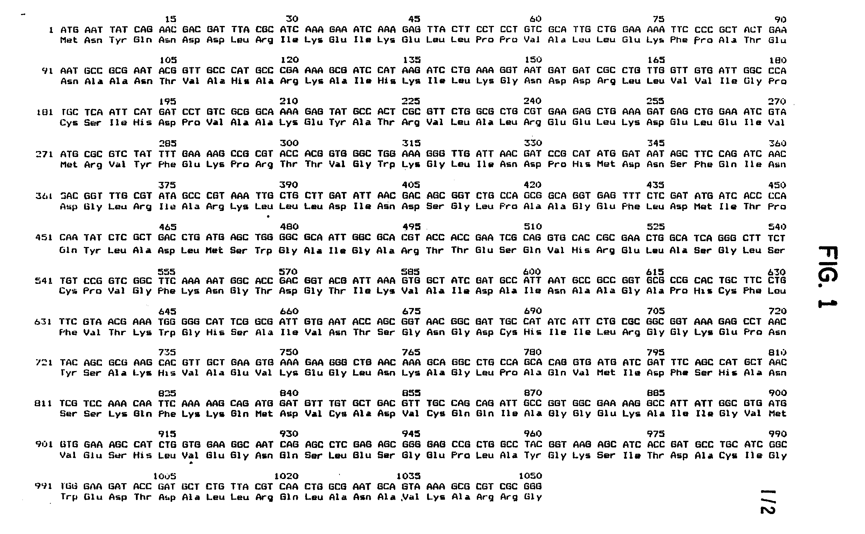

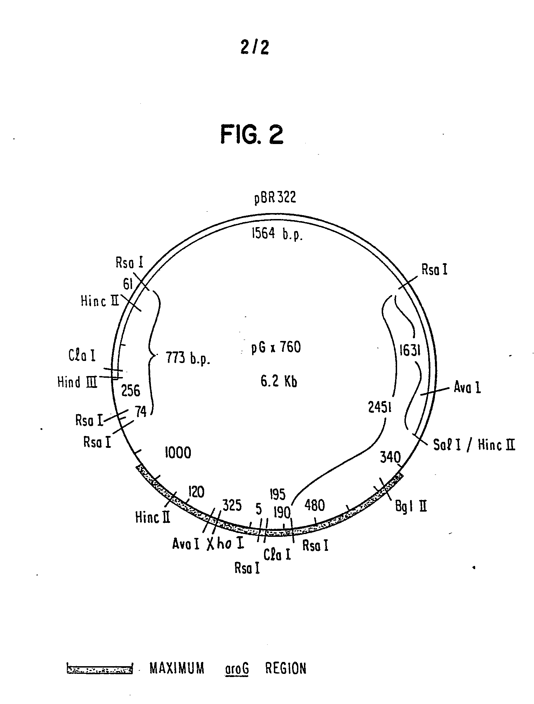

The size of the plasmid insertion was further reduced by sequential restriction enzyme digestions. First, the plasmid was subjected to a partial digestion with the enzyme Sau3A which resulted in the deletion of approximately 3.7 kb from the insert, yet resulted in cells having the feedback resistant aroG activity. The resultant plasmid was further digested with a mixture of Hind III and Sal I resulting in a deletion of approximately 1.5 kb to yield a plasmid having an insertion of about 2.7 kb and having the desired DAHP synthetase activity. A sample of this strain was deposited with the American Type Culture Collection, Rockville, Maryland, as ATCC 31992. The nucleotide sequence of the DAHP synthetase gene in this plasmid was determined and is shown in Figure 1 of the drawings. The plasmid is illustrated in Figure 2 of the drawings. The DAHP synthetase gene (aroG) extends from approximately the Bgl II site to the Hinc II site, which is the direction of transcription.

CLAIMS

1.  A substantially isolated intact microbial gene that specifies the biosynthesis of a 3-deoxy-2-keto-D-arabinoheptulosonic acid-7-phosphate (DAHP) synthetase, which is resistant to feedback inhibition by aromatic amino acids.

2.  The microbial gene of claim 1, comprising the following deoxyribonucleotide sequence:

```
1                                        15
A T G   A A Y   T A Y   C A M   A A Y
Met     Asn     Tyr     Gln     Asn

                                         30
G A Y   G A Y   Y T Z   L G N   A T H
Asp     Asp     Leu     Arg     Ile

                                         45
5  A A M   G A M   A T H   A A M   G A M
   Lys     Glu     Ile     Lys     Glu

                                         60
   Y T Z   Y T Z   C C X   C C X   G T X
   Leu     Leu     Pro     Pro     Val

                                         75
   G C X   Y T Z   Y T Z   G A M   A A M
10 Ala     Leu     Leu     Glu     Lys

                                         90
   T T Y   C C X   G C X   A C X   G A M
   Phe     Pro     Ala     Thr     Glu

                                         105
   A A Y   G C X   G C X   A A Y   A C X
   Asn     Ala     Ala     Asn     Thr

                                         120
15 G T X   G C X   C A Y   G C X   L G N
   Val     Ala     His     Ala     Arg

                                         135
   A A M   G C X   A T H   C A Y   A A M
   Lys     Ala     Ile     His     Lys

                                         150
   A T H   Y T Z   A A M   G G X   A A Y
20 Ile     Leu     Lys     Gly     Asn

                                         165
   G A Y   G A Y   L G N   Y T Z   Y T Z
   Asp     Asp     Arg     Leu     Leu

                                         170
   G T X   G T X   A T H   G G X   C C X
   Val     Val     Ile     Gly     Pro

                                         195
25 T G Y   Q R S   A T H   C A Y   G A Y
   Cys     Ser     Ile     His     Asp
```

| | | | | 210 |
|---|---|---|---|---|
| C C X | G T X | G C X | G C X | A A M |
| Pro | Val | Ala | Ala | Lys |

| | | | | 225 |
|---|---|---|---|---|
| G A M | T A Y | G C X | A C X | L G N |
| Glu | Tyr | Ala | Thr | Arg |

| | | | | 240 |
|---|---|---|---|---|
| G T X | Y T Z | G C X | Y T Z | L G N |
| Val | Leu | Ala | Leu | Arg |

| | | | | 255 |
|---|---|---|---|---|
| G A M | G A M | Y T Z | A A M | G A Y |
| Glu | Glu | Leu | Lys | Asp |

| | | | | 270 |
|---|---|---|---|---|
| G A M | Y T Z | G A M | A T H | G T X |
| Glu | Leu | Glu | Ile | Val |

| | | | | 285 |
|---|---|---|---|---|
| A T G | L G N | G T X | T A Y | T T Y |
| Met | Arg | Val | Tyr | Phe |

| | | | | 300 |
|---|---|---|---|---|
| G A M | A A M | C C X | L G N | A C X |
| Glu | Lys | Pro | Arg | Thr |

| | | | | 315 |
|---|---|---|---|---|
| A C X | G T X | G G X | T G G | A A M |
| Thr | Val | Gly | Trp | Lys |

| | | | | 330 |
|---|---|---|---|---|
| G G X | Y T Z | A T H | A A Y | G A Y |
| Gly | Leu | Ile | Asn | Asp |

| | | | | 345 |
|---|---|---|---|---|
| C C X | C A Y | A T G | G A Y | A A Y |
| Pro | His | Met | Asp | Asn |

| | | | | 360 |
|---|---|---|---|---|
| Q R S | T T Y | C A M | A T H | A A Y |
| Ser | Phe | Gln | Ile | Asn |

| | | | | 375 |
|---|---|---|---|---|
| G A Y | G G X | Y T Z | L G N | A T H |
| Asp | Gly | Leu | Arg | Ile |

| | | | | 390 |
|---|---|---|---|---|
| G C X | L G N | A A M | Y T Z | Y T Z |
| Ala | Arg | Lys | Leu | Leu |

| | | | | 405 |
|---|---|---|---|---|
| Y T Z | G A Y | A T H | A A Y | G A Y |
| Leu | Asp | Ile | Asn | Asp |

```
                                                          420
    Q R S    G G X    Y T Z    C C X    G C X
     Ser      Gly      Leu      Pro      Ala

                                                          435
    G C X    G G X    G A M    T T Y    Y T Z
     Ala      Gly      Glu      Phe      Leu

                                                          450
5   G A Y    A T G    A T H    A C X    C C X
     Asp      Met      Ile      Thr      Pro

                                                          465
    C A M    T A Y    Y T Z    G C X    G A Y
     Gln      Tyr      Leu      Ala      Asp

                                                          480
10  Y T Z    A T G    Q R S    T G G    G G X
     Leu      Met      Ser      Trp      Gly

                                                          495
    G C X    A T H    G G X    G C X    L G N
     Ala      Ile      Gly      Ala      Arg

                                                          510
    A C X    A C X    G A M    Q R S    C A M
     Thr      Thr      Glu      Ser      Gln

                                                          525
15  G T X    C A Y    L G N    G A M    Y T Z
     Val      His      Arg      Glu      Leu

                                                          540
    G C X    Q R S    G G X    Y T Z    Q R S
     Ala      Ser      Gly      Leu      Ser
                                                          555
    T G Y    C C X    G T X    G G X    T T Y
20   Cys      Pro      Val      Gly      Phe

                                                          570
    A A M    A A Y    G G X    A C X    G A Y
     Lys      Asn      Gly      Thr      Asp

                                                          585
    G G X    A C X    A T H    A A M    G T X
     Gly      Thr      Ile      Lys      Val

                                                          600
25  G C X    A T H    G A Y    G C X    A T H
     Ala      Ile      Asp      Ala      Ile

                                                          615
    A A Y    G C X    G C X    G G X    G C X
     Asn      Ala      Ala      Gly      Ala
```

```
                                             630
      C C X    C A Y    T G Y    T T Y    Y T Z
      Pro      His      Cys      Phe      Leu

                                             645
      T T Y    G T X    A C X    A A M    T G G
      Phe      Val      Thr      Lys      Trp

                                             660
      G G X    C A Y    Q R S    G C X    A T H
      Gly      His      Ser      Ala      Ile

                                             675
      G T X    A A Y    A C X    Q R S    G G X
      Val      Asn      Thr      Ser      Gly

                                             690
      A A Y    G G X    G A Y    T Q Y    C A Y
      Asn      Gly      Asp      Cys      His

                                             705
      A T H    A T H    Y T Z    L G N    G G X
      Ile      Ile      Leu      Arg      Gly

                                             720
      G G X    A A M    G A M    C C X    A A Y
      Gly      Lys      Glu      Pro      Asn

                                             735
      T A Y    Q R S    G C X    A A M    C A Y
      Tyr      Ser      Ala      Lys      His

                                             750
      G T X    G C X    G A M    G T X    A A M
      Val      Ala      Glu      Val      Lys

                                             765
      G A M    G G X    Y T Z    A A Y    A A M
      Glu      Gly      Leu      Asn      Lys

                                             780
      G C X    G G X    Y T Z    C C X    G C X
      Ala      Gly      Leu      Pro      Ala

                                             795
      C A M    G T X    A T G    A T H    G A Y
      Gln      Val      Met      Ile      Asp

                                             810
      T T Y    Q R S    C A Y    G C X    A A Y
      Phe      Ser      His      Ala      Asn

                                             825
      Q R S    Q R S    A A M    C A M    T T Y
      Ser      Ser      Lys      Gln      Phe
```

0077196

- 18 -

```
                                              840
   A A M   A A M   C A M   A T G   G A Y
   Lys     Lys     Gln     Met     Asp

                                              855
   G T X   T G Y   G C X   G A Y   G T X
   Val     Cys     Ala     Asp     Val

                                              870
   T G Y   C A M   C A M   A T H   G C X
   Cys     Gln     Gln     Ile     Ala

                                              885
   G G X   G G X   G A M   A A M   G C X
   Gly     Gly     Glu     Lys     Ala

                                              900
   A T H   A T H   G G X   G T X   A T G
   Ile     Ile     Gly     Val     Met
                                              915
   Val     Glu     Ser     His     Leu
   G T X   G A M   Q R S   C A Y   Y T Z

                                              930
   Val     Glu     Gly     Asn     Gln
   G T X   G A M   G G X   A A Y   C A M

                                              945
   Ser     Leu     Glu     Ser     Gly
   Q R S   Y T Z   G A M   Q R S   G G X

                                              960
   Glu     Pro     Leu     Ala     Tyr
   G A M   C C X   Y T Z   G C X   T A Y

                                              975
   Gly     Lys     Ser     Ile     Thr
   G G X   A A M   Q R S   A T H   A C X

                                              990
   Asp     Ala     Cys     Ile     Gly
   G A Y   G C X   T G Y   A T H   G G X

                                             1005
   Trp     Glu     Asp     Thr     Asp
   T G G   G A M   G A Y   A C X   G A Y

                                             1020
   Ala     Leu     Leu     Arg     Gln
   G C X   Y T Z   Y T Z   L G N   C A M

                                             1035
   Leu     Ala     Asn     Ala     Val
   Y T Z   G C X   A A Y   G C X   G T X
```

```
                                       1050
     Lys      Ala     Arg     Arg     Gly
     A A M   G C X   L G N   L G N   G G X
```

wherein, the 5' to 3' strand, beginning with the amine terminus and the amino acids for which each triplet codes are shown, and wherein within each triplet,

A is deoxyadenyl

T is thymidyl

G is deoxyguanyl

C is deoxycytosyl

X is A, T, C or G

Y is T or C

When Y is C, Z is A or G

H is A, T or C

Q is T or A When Q is T, R is C and S is A, T, C or G

When Q is A, R is G and S is T or C

M is A or G

L is A or C

When L is A, N is A or G

When L is C, N is A, T, C or G.

Gly is glycine

Ala is alanine

Val is valine

Leu is leucine

Ile is isoleucine

Ser is serine

Thr is threonine Phe is phenylalanine

Tyr is tyrosine

Trp is tryptophan

Cys is cysteine

Met is methionine

Asp is aspartic acid Glu is gluatmic acid

Lys is lysine

ARg is arginine

0077196

His is histidine
Pro is proline
Gln is glutamine
Asn is asparagine

3.    The microbial gene of claim 1,
comprising the following deoxyribonucleotide sequence:

| | | | | |
|---|---|---|---|---|
| 1 ATG Met | AAT Asn | TAT Tyr | CAG Gln | 15 AAC Asn |
| GAC Asp | GAT Asp | TTA Leu | CGC Arg | 30 ATC Ile |
| 5 AAA Lys | GAA Glu | ATC Ile | AAA Lys | 45 GAG Glu |
| TTA Leu | CTT Leu | CCT Pro | CCT Pro | 60 GTC Val |
| 10 GCA Ala | TTG Leu | CTG Leu | GAA Glu | 75 AAA Lys |
| TTC Phe | CCC Pro | GCT Ala | ACT Thr | 90 GAA Glu |
| AAT Asn | GCC Ala | GCG Ala | AAT Asn | 105 ACG Thr |
| 15 GTT Val | GCC Ala | CAT His | GCC Ala | 120 CGA Arg |
| AAA Lys | GCG Ala | ATC Ile | CAT His | 135 AAG Lys |
| 20 ATC Ile | CTG Leu | AAA Lys | GGT Gly | 150 AAT Asn |
| GAT Asp | GAT Asp | CGC Arg | CTG Leu | 165 TTG Leu |
| GTT Val | GTG Val | ATT Ile | GGC Gly | 180 CCA Pro |

|   |   |   | | 195 |
| TGC | TCA | ATT | CAT | GAT |
| Cys | Ser | Ile | His | Asp |

|   |   |   | | 210 |
| CCT | GTC | GCG | GCA | AAA |
| Pro | Val | Ala | Ala | Lys |

|   |   |   | | 225 |
| GAG | TAT | GCC | ACT | CGC |
| Glu | Tyr | Ala | Thr | Arg |

|   |   |   | | 240 |
| GTT | CTG | GCG | CTG | CGT |
| Val | Leu | Ala | Leu | Arg |

|   |   |   | | 255 |
| GAA | GAG | CTG | AAA | GAT |
| Glu | Glu | Leu | Lys | Asp |

|   |   |   | | 270 |
| GAG | CTG | GAA | ATC | GTA |
| Glu | Leu | Glu | Ile | Val |

|   |   |   | | 285 |
| ATG | CGC | GTC | TAT | TTT |
| Met | Arg | Val | Tyr | Phe |

|   |   |   | | 300 |
| GAA | AAG | CCG | CGT | ACC |
| Glu | Lys | Pro | Arg | Thr |

|   |   |   | | 315 |
| ACG | GTG | GGC | TGG | AAA |
| Thr | Val | Gly | Trp | Lys |

|   |   |   | | 330 |
| GGG | TTG | ATT | AAC | GAT |
| Gly | Leu | Ile | Asn | Asp |

|   |   |   | | 345 |
| CCG | CAT | ATG | GAT | AAT |
| Pro | His | Met | Asp | Asn |

|   |   |   | | 360 |
| AGC | TTC | CAG | ATC | AAC |
| Ser | Phe | Gln | Ile | Asn |

|       |       |       |       | 375 |
|-------|-------|-------|-------|-----|
| G A C | G G T | T T G | C G T | A T A |
| Asp   | Gly   | Leu   | Arg   | Ile |

|       |       |       |       | 390 |
|-------|-------|-------|-------|-----|
| G C C | C G T | A A A | T T G | C T G |
| Ala   | Arg   | Lys   | Leu   | Leu |

|       |       |       |       | 405 |
|-------|-------|-------|-------|-----|
| C T T | G A T | A T T | A A C | G A C |
| Leu   | Asp   | Ile   | Asn   | Asp |

|       |       |       |       | 420 |
|-------|-------|-------|-------|-----|
| A G C | G G T | C T G | C C A | G C G |
| Ser   | Gly   | Leu   | Pro   | Ala |

|       |       |       |       | 435 |
|-------|-------|-------|-------|-----|
| G C A | G G T | G A G | T T T | C T C |
| Ala   | Gly   | Glu   | Phe   | Leu |

|       |       |       |       | 450 |
|-------|-------|-------|-------|-----|
| G A T | A T G | A T C | A C C | C C A |
| Asp   | Met   | Ile   | Thr   | Pro |

|       |       |       |       | 465 |
|-------|-------|-------|-------|-----|
| C A A | T A T | C T C | G C T | G A C |
| Gln   | Tyr   | Leu   | Ala   | Asp |

|       |       |       |       | 480 |
|-------|-------|-------|-------|-----|
| C T G | A T G | A G C | T G G | G G C |
| Leu   | Met   | Ser   | Trp   | Gly |

|       |       |       |       | 495 |
|-------|-------|-------|-------|-----|
| G C A | A T T | G G C | G C A | C G T |
| Ala   | Ile   | Gly   | Ala   | Arg |

|       |       |       |       | 510 |
|-------|-------|-------|-------|-----|
| A C C | A C C | G A A | T C G | C A G |
| Thr   | Thr   | Glu   | Ser   | Gln |

|       |       |       |       | 525 |
|-------|-------|-------|-------|-----|
| G T G | C A C | C G C | G A A | C T G |
| Val   | His   | Arg   | Glu   | Leu |

|       |       |       |       | 540 |
|-------|-------|-------|-------|-----|
| G C A | T C A | G G G | C T T | T C T |
| Ala   | Ser   | Gly   | Leu   | Ser |

|      |     |     |     |     | 555 |
|------|-----|-----|-----|-----|-----|
| | T G T | C C G | G T C | G G C | T T C |
| | Cys | Pro | Val | Gly | Phe |
| | | | | | 570 |
| | A A A | A A T | G G C | A C C | G A C |
| | Lys | Asn | Gly | Thr | Asp |
| | | | | | 585 |
| | G G T | A C G | A T T | A A A | G T G |
| | Gly | Thr | Ile | Lys | Val |
| | | | | | 600 |
| | G C T | A T C | G A T | G C C | A T T |
| | Ala | Ile | Asp | Ala | Ile |
| | | | | | 615 |
| | A A T | G C C | G C C | G G T | G C G |
| | Asn | Ala | Ala | Gly | Ala |
| | | | | | 630 |
| | C C G | C A C | T G C | T T C | C T G |
| | Pro | His | Cys | Phe | Leu |
| | | | | | 645 |
| | T T C | G T A | A C G | A A A | T G G |
| | Phe | Val | Thr | Lys | Trp |
| | | | | | 660 |
| | G G G | C A T | T C G | G C G | A T T |
| | Gly | His | Ser | Ala | Ile |
| | | | | | 675 |
| | G T G | A A T | A C C | A G C | G G T |
| | Val | Asn | Thr | Ser | Gly |
| | | | | | 690 |
| | A A C | G G C | G A T | T G C | C A T |
| | Asn | Gly | Asp | Cys | His |
| | | | | | 705 |
| | A T C | A T T | C T G | C G C | G G C |
| | Ile | Ile | Leu | Arg | Gly |
| | | | | | 720 |
| | G G T | A A A | G A G | C C T | A A C |
| | Gly | Lys | Glu | Pro | Asn |

|  |  |  |  | 735 |
| --- | --- | --- | --- | --- |
| T A C | A G C | G C G | A A G | C A C |
| Tyr | Ser | Ala | Lys | His |
|  |  |  |  | 750 |
| G T T | G C T | G A A | G T G | A A A |
| Val | Ala | Glu | Val | Lys |
|  |  |  |  | 765 |
| G A A | G G G | C T G | A A C | A A A |
| Glu | Gly | Leu | Asn | Lys |
|  |  |  |  | 780 |
| G C A | G G C | C T G | C C A | G C A |
| Ala | Gly | Leu | Pro | Ala |
|  |  |  |  | 795 |
| C A G | G T G | A T G | A T C | G A T |
| Gln | Val | Met | Ile | Asp |
|  |  |  |  | 810 |
| T T C | A G C | C A T | G C T | A A C |
| Phe | Ser | His | Ala | Asn |
|  |  |  |  | 825 |
| T C G | T C C | A A A | C A A | T T C |
| Ser | Ser | Lys | Gln | Phe |
|  |  |  |  | 840 |
| A A A | A A G | C A G | A T G | G A T |
| Lys | Lys | Gln | Met | Asp |
|  |  |  |  | 855 |
| G T T | T G T | G C T | G A C | G T T |
| Val | Cys | Ala | Asp | Val |
|  |  |  |  | 870 |
| T G C | C A G | C A G | A T T | G C C |
| Cys | Gln | Gln | Ile | Ala |
|  |  |  |  | 885 |
| G G T | G G C | G A A | A A G | G C C |
| Gly | Gly | Glu | Lys | Ala |
|  |  |  |  | 900 |
| A T T | A T T | G G C | G T G | A T G |
| Ile | Ile | Gly | Val | Met |

|       |            |            |            |            | 915        |
|-------|------------|------------|------------|------------|------------|
|       | G T G      | G A A      | A G C      | C A T      | C T G      |
|       | Val        | Glu        | Ser        | His        | Leu        |
|       |            |            |            |            | 930        |
|       | G T G      | G A A      | G G C      | A A T      | C A G      |
|       | Val        | Glu        | Gly        | Asn        | Gln        |
|       |            |            |            |            | 945        |
|       | A G C      | C T C      | G A G      | A G C      | G G G      |
|       | Ser        | Leu        | Glu        | Ser        | Gly        |
|       |            |            |            |            | 960        |
|       | G A G      | C C G      | C T G      | G C C      | T A C      |
|       | Glu        | Pro        | Leu        | Ala        | Tyr        |
|       |            |            |            |            | 975        |
|       | G G T      | A A G      | A G C      | A T C      | A C C      |
|       | Gly        | Lys        | Ser        | Ile        | Thr        |
|       |            |            |            |            | 990        |
|       | G A T      | G C C      | T G C      | A T C      | G G C      |
|       | Asp        | Ala        | Cys        | Ile        | Gly        |
|       |            |            |            |            | 1005       |
|       | T G G      | G A A      | G A T      | A C C      | G A T      |
|       | Trp        | Glu        | Asp        | Thr        | Asp        |
|       |            |            |            |            | 1020       |
|       | G C T      | C T G      | T T A      | C G T      | C A A      |
|       | Ala        | Leu        | Leu        | Arg        | Gln        |
|       |            |            |            |            | 1035       |
|       | C T G      | G C G      | A A T      | G C A      | G T A      |
|       | Leu        | Ala        | Asn        | Ala        | Val        |
|       |            |            |            |            | 1050       |
|       | A A A      | G C G      | C G T      | C G C      | G G G      |
|       | Lys        | Ala        | Arg        | Arg        | Gly        |

4.   The gene of claim 1, wherein the gene is a feedback resistant <u>aroG</u>, <u>aroF</u> or <u>aroH</u> gene of bacterial origin.

5.   A plasmid capable of replication in a bacterial cell, comprising the gene of claims 1, 2, 3 or 4.

6.   The plasmid of claim 5, comprising the bacterial plasmid pMF-3 or pBR322.

7.   A microorganism transformed with the plasmid of claim 5, having the ability to maintain and replicate said plasmid.

8.   The microorganism of claim 7, wherein said plasmid includes signals recognizable by said microorganism which control the expression of the feedback resistant DAHP synthetase gene, whereby said microorganism has the ability to produce DAHP synthetase which is active in the presence of normally inhibiting amounts of aromatic amino acids.

9.   A method for cloning a DNA segment which comprises first, inserting said segment into a single-copy plasmid to produce a first recombinant plasmid, transforming microbial cells with said first recombinant plasmid, isolating plasmid DNA from said microbial cells and excising said DNA segment or a portion thereof from said plasmid DNA and, second, inserting said excised segment into a multi-copy plasmid to form a second recombinant plasmid and transforming microbial cells with said second recombinant plasmid.

10.   The method of claim 9 wherein the DNA segment to be cloned contains a feedback resistant DAHP synthetase gene.

0077196

11.   A method of producing an aromatic amino acid comprising culturing a microorganism as claimed in claim 8 and recovering said amino acid.

**FIG. 1**

```
              15             30             45             60             75             90
  1 ATG AAT TAT CAG AAC GAC GAT TTA CGC ATC AAA GAA ATC AAA GAG TTA CTT CCT CCT GTC GCA TTG CTG GAA AAA TTC CCC GCT ACT GAA
    Met Asn Tyr Gln Asn Asp Asp Leu Arg Ile Lys Glu Ile Lys Glu Leu Leu Pro Pro Val Ala Leu Leu Glu Lys Phe Pro Ala Thr Glu

              105            120            135            150            165            180
 91 AAT GCC GCG AAT ACG GTT GCC CAT GCC CGA AAA GCG ATC CAT AAG ATC CTG AAA GGT AAT GAT GAT CGC CTG TTG GTT GTG ATT GGC CCA
    Asn Ala Ala Asn Thr Val Ala His Ala Arg Lys Ala Ile His Lys Ile Leu Lys Gly Asn Asp Asp Arg Leu Leu Val Val Ile Gly Pro

              195            210            225            240            255            270
181 TGC TCA ATT CAT GAT CCT GTC GCG GCA AAA GAG TAT GCC ACT CGC GTT CTG GCG CTG CGT GAA GAG CTG AAA GAT GAG CTG GAA ATC GTA
    Cys Ser Ile His Asp Pro Val Ala Ala Lys Glu Tyr Ala Thr Arg Val Leu Ala Leu Arg Glu Glu Leu Lys Asp Glu Leu Glu Ile Val

              285            300            315            330            345            360
271 ATG CGC GTC TAT TTT GAA AAG CCG CGT ACC ACG GTG GGC TGG AAA GGG TTG ATT AAC GAT CCG CAT ATG GAT AAT AGC TTC CAG ATC AAC
    Met Arg Val Tyr Phe Glu Lys Pro Arg Thr Thr Val Gly Trp Lys Gly Leu Ile Asn Asp Pro His Met Asp Asn Ser Phe Gln Ile Asn

              375            390            405            420            435            450
361 GAC GGT TTG CGT ATA GCC CGT AAA TTG CTG CTT GAT ATT AAC GAC AGC GGT CTG CCA GCG GCA GGT GAG TTT CTC GAT ATG ATC ACC CCA
    Asp Gly Leu Arg Ile Ala Arg Lys Leu Leu Leu Asp Ile Asn Asp Ser Gly Leu Pro Ala Ala Gly Glu Phe Leu Asp Met Ile Thr Pro

              465            480            495            510            525            540
451 CAA TAT CTC GCT GAC CTG ATG AGC TGG GGC GCA ATT GGC GCA CGT ACC ACC GAA TCG CAG GTG CAC CGC GAA CTG GCA TCA GGG CTT TCT
    Gln Tyr Leu Ala Asp Leu Met Ser Trp Gly Ala Ile Gly Ala Arg Thr Thr Glu Ser Gln Val His Arg Glu Leu Ala Ser Gly Leu Ser

              555            570            585            600            615            630
541 TGT CCG GTC GGC TTC AAA AAT GGC ACC GAC GGT ACG ATT AAA GTG GCT ATC GAT GCC ATT AAT GCC GCC GGT GCG CCG CAC TGC TTC CTG
    Cys Pro Val Gly Phe Lys Asn Gly Thr Asp Gly Thr Ile Lys Val Ala Ile Asp Ala Ile Asn Ala Ala Gly Ala Pro His Cys Phe Leu

              645            660            675            690            705            720
631 TTC GTA ACG AAA TGG GGG CAT TCG GCG ATT GTG AAT ACC AGC GGT AAC GGC GAT TGC CAT ATC ATT CTG CGC GGC GGT AAA GAG CCT AAC
    Phe Val Thr Lys Trp Gly His Ser Ala Ile Val Asn Thr Ser Gly Asn Gly Asp Cys His Ile Ile Leu Arg Gly Gly Lys Glu Pro Asn

              735            750            765            780            795            810
721 TAC AGC GCG AAG CAC GTT GCT GAA GTG AAA GAA GGG CTG AAC AAA GCA GGC CTG CCA GCA CAG GTG ATG ATC GAT TTC AGC CAT GCT AAC
    Tyr Ser Ala Lys His Val Ala Glu Val Lys Glu Gly Leu Asn Lys Ala Gly Leu Pro Ala Gln Val Met Ile Asp Phe Ser His Ala Asn

              825            840            855            870            885            900
811 TCG TCC AAA CAA TTC AAA AAG CAG ATG GAT GTT TGT GCT GAC GTT TGC CAG CAG ATT GCC GGT GGC GAA AAG GCC ATT ATT GGC GTG ATG
    Ser Ser Lys Gln Phe Lys Lys Gln Met Asp Val Cys Ala Asp Val Cys Gln Gln Ile Ala Gly Gly Glu Lys Ala Ile Ile Gly Val Met

              915            930            945            960            975            990
901 GTG GAA AGC CAT CTG GTG GAA GGC AAT CAG AGC CTC GAG AGC GGG GAG CCG CTG GCC TAC GGT AAG AGC ATC ACC GAT GCC TGC ATC GGC
    Val Glu Ser His Leu Val Glu Gly Asn Gln Ser Leu Glu Ser Gly Glu Pro Leu Ala Tyr Gly Lys Ser Ile Thr Asp Ala Cys Ile Gly

              1005           1020           1035           1050
991 TGG GAA GAT ACC GAT GCT CTG TTA CGT CAA CTG GCG AAT GCA GTA AAA GCG CGT CGC GGG
    Trp Glu Asp Thr Asp Ala Leu Leu Arg Gln Leu Ala Asn Ala Val Lys Ala Arg Arg Gly
```

1/2

2/2

FIG. 2

MAXIMUM  aroG  REGION